# EUROPÄISCHE PATENTSCHRIFT

(11) **EP 0 910 276 B1**
(45) Veröffentlichungstag und Bekanntmachung des Hinweises auf die Patenterteilung: **08.09.2004**
(21) Anmeldenummer: 98909407.3
(22) Anmeldetag: 06.02.1998
(51) Int. Cl.: A61B 1/00

(54) **KUPPLUNG ZUM DICHTEN VERBINDEN ZWEIER SCHAFTARTIGER MEDIZINISCHER INSTRUMENTE**
COUPLING FOR SEALINGLY CONNECTING TWO ELONGATE MEDICAL INSTRUMENTS
ELEMENT D'ACCOUPLEMENT ETANCHE DE DEUX INSTRUMENTS MEDICAUX ALLONGES

(30) Priorität: 07.02.1997 DE 19704579
(43) Veröffentlichungstag der Anmeldung: 28.04.1999
(73) Patentinhaber: Karl Storz GmbH & Co. KG, 78532 Tuttlingen (DE)
(72) Erfinder: DITTRICH, Horst, D-78194 Immendingen (DE); BACHER, Uwe, D-78532 Tuttlingen (DE)
(74) Vertreter: Weller, Wolfgang, Dr.rer.nat.
(86) Internationale Anmeldenummer: PCT/EP1998/000668
(87) Internationale Veröffentlichungsnummer: WO 1998/034531

(56) Entgegenhaltungen:
- DE-A- 4 018 578
- DE-A- 4 425 705
- DE-U- 29 602 142

## Beschreibung

Die Erfindung betrifft eine Kupplung zum dichten Verbinden zweier schaftartiger medizinischer Instrumente längs deren Schaftachse, mit einem an einem ersten Instrument angeordneten, eine Anlagefläche aufweisendes Kupplungselement, an dessen Anlagefläche eine Anlagefläche eines Kupplungselementes des zweiten Instrumentes längs einer Kupplungsachse anlegbar ist, und mit einer Verriegelung zum lösbaren Verriegeln der beiden Kupplungselemente miteinander, wobei die Anlageflächen dichtend aneinanderliegen.

Eine derartige Kupplung ist bspw. aus dem Firmenprospekt der Anmelderin "STORZ, DIE WELT DER ENDOSKOPIE", Arthroskopie 4. Auflage 1/90, bekannt. Diese Kupplung dient zum Verbinden eines Arthroskopschaftes mit einer Optik.

Insbesondere in der zwischenzeitlich weit verbreiteten minimalinvasiven Chirurgie finden im Laufe einer Untersuchung oder einer Operation zahlreiche Vorgänge statt, bei denen zwei medizinische Instrumente miteinander verbunden und auch wieder gelöst werden müssen.

Üblicherweise werden bei dieser Operationstechnik mehrere Hohlschäfte über Trokare in den Körper eingeführt, und anschließend werden durch den Innenraum des Hohlschaftes medizinische Instrumente in den Körper eingeführt, die ebenfalls schaftartige Körper aufweisen.

Bei manchen Vorgängen ist es erforderlich, die beiden medizinischen Instrumente miteinander zu verbinden, wozu die eingangs genannte Kupplung notwendig ist. Da über einen in den menschlichen Körper eingeführten hohlen Schaft Körperflüssigkeiten oder Körpergase austreten können, ist es bei an einen solchen Schaft angekoppelten weiteren Instrumenten notwendig, daß diese Verbindung dichtend erfolgt.

Aus dem eingangs genannten Firmenprospekt ist bspw. auf der Seite mit der Bezeichnung "ART-LJ 3" ein Arthroskopschaft ersichtlich, in den eine auf der Seite "ART-LJ 2" ersichtliche Optik eingeschoben und mit dem Arthroskopschaft verbunden werden soll.

Ein solcher Verbund aus den beiden miteinander verkuppelten medizinischen Instrumenten, nämlich Arthroskopschaft und Optik, ist aus dem vorgenannten Firmenprospekt auf der einleitenden Seite des Kapitels "Arthroskopisches Drainagesytem nach Christensen" oder auf der Seite mit der Bezeichnung "ART-OP-INST 19" ff. beschrieben.

Aus dieser Beschreibung der operativen Technik einer arthroskopischen Kreuzbandplastik ist ersichtlich, daß häufige Wechselvorgänge der miteinander verbundenen Instrumente, nämlich Arthroskopschaft und Optik, notwendig sind.

Die aus diesem Prospekt bekannte Kupplung weist eine Verriegelung auf, die auf dem Bajonettprinzip beruht.

Eines der Instrumente, bspw. der Arthroskopschaft, weist eine Hülse mit einer in deren Wand eingeschnittene Bajonettführung auf, in die eine oder bei einer doppelten Bajonettführung zwei entsprechend ausgebildete diametral gegenüberliegende Nasen eines Kupplungselementes der Optik eingeführt werden müssen. Durch Verdrehen der beiden zu kuppelnden Instrumente relativ zueinander wird die mechanische Verriegelung gegenüber Abziehen längs der Kupplungsachse, die sich in der Längsachse der Schäfte der Instrumente erstreckt, erreicht. Durch die Steigung der Bajonettwindung werden die Instrumente längs der Kupplungsachse etwas aufeinander zu bewegt, so daß die entsprechenden Anlageflächen dichtend aneinander gepreßt werden.

Diese Kupplungsprinzip weist nun einige Nachteile auf.

Ein erster Nachteil besteht darin, daß eine hohe Aufmerksamkeit beim Aneinanderfügen der beiden Instrumente notwendig ist, da die Nasen des einen Kupplungselementes nur in einer ganz bestimmten Drehstellung in die Bajonettführung des anderen Instrumentes eingesetzt werden können.

Ein weiterer Nachteil besteht darin, daß beim Verbindungsvorgang die beiden schaftartigen Instrumente relativ zueinander verdreht werden können, wozu jedes der beiden Instrumente von einer anderen Hand ergriffen werden muß. Es ist zu berücksichtigen, daß meist eines der beiden Instrumente bereits in einen menschlichen Körper eingeführt ist, so daß ein Verdrehen nur des an dieses Instrument anzukuppelnden anderen Instruments die Gefahr beinhaltet, daß auch das bereits im Körper steckende Instrument verdreht wird, was zu Schädigungen oder Verletzungen des Patienten führen kann.

Betrachtet man den im vorgenannten Firmenprospekt auf der Seite mit der Bezeichnung "ART-OP-INST 19" gezeigten Zusammenbau, so ist ersichtlich, daß von der an den Arthroskopschaft angekoppelten Optik mehrere, ggf. meterlange, zu Monitoren oder sonstigen Gerätschaften führende Leitungen seitlich abstehen. Wie zuvor erwähnt, ist konstruktionsbedingt bei einem Bajonettverschluß, vor und nach Bewerkstelligung des Kupplungsvorgangs, eine ganz bestimmte Relativstellung zwischen den beiden Instrumenten vorgegeben. Diese Stellung kann aber bspw. für den Operateur ungünstig sein und dessen Bewegungsfreiheit einschränken. Ungünstig abstehende Bauteile können auf den Zusammenbau ein Drehmoment ausüben, was unerwünscht ist und dazu führt, daß der Zusammenbau vom Operateur angestrengt in einer bestimmten Drehlage gehalten werden muß.

Es ist daher Aufgabe der vorliegenden Erfindung, eine Kupplung der eingangs genannten Art dahingehend weiterzuentwickeln, daß der Kupplungsvorgang ohne hohe Aufmerksamkeit und insbesondere nur mit einer Hand durchgeführt werden kann, und durch eine verbesserte Handhabbarkeit und Betriebssicherheit außerdem das Beeinträchtigungsrisiko für den Patienten erheblich gemindert ist.

Erfindungsgemäß wird die Aufgabe dadurch gelöst, daß die Verriegelung einen quer zur Kupplungsachse verschiebbaren Schieber aufweist, der an einem der beiden Kupplungselemente querverschiebbar angeordnet ist und der einen größeren Öffnungsbereich und einen kleineren Öffnungsbereich aufweist, wobei der verschiebbare Schieber in einer ersten Schiebestellung das Anlegen der beiden Kupplungselemente durch den größeren Öffnungsbereich aneinander ermöglicht und in einer zweiten riegelnden Schiebestellung die beiden Kupplungselemente in dem kleineren Öffnungsbereich mechanisch verriegelt, und daß ein die Anlagefläche aufweisendes Bauteil zumindest eines Kupplungselements in Richtung der Kupplungsachse verschiebbar ist.

Durch die erste Merkmalskombination bezüglich der Ausgestaltung des Schiebers wird die Möglichkeit eröffnet, daß das an das eine medizinische Instrument anzukuppelnde zweite Instrument, in der ersten nicht verriegelnden Schiebestellung des Schiebers angesetzt werden kann und durch Verschieben des Schiebers quer zur Längsachse bzw. zur Kupplungsachse die beiden Kupplungselemente der beiden Instrumente miteinander mechanisch verriegelt werden. Die beiden Elemente müssen lediglich aneinandergefügt werden, was bspw. durch Ertasten mit einer Hand einfach durchgeführt werden kann. Mit derselben Hand kann dann der querverschiebbare Schieber bewegt werden, ohne daß dazu die aneinandergefügten Teile relativ zueinander verdreht werden müssen. Somit ist dieser Vorgang einfach und ohne hohe Aufmerksamkeit und außerdem mit nur einer Hand durchzuführen.

Aufgrund der Tatsache, daß zumindest ein Bauteil eines Kupplungselementes in Richtung der Kupplungsachse verschiebbar ist, kann durch Verschieben der aneinandergefügten Instrumente längs der Kupplungsachse der notwendige Anpreßdruck zwischen den Anlageflächen geschaffen werden, um einen dichten Abschluß zu schaffen. Die Verschiebung kann bspw. durch den querverschiebbaren Schieber verursacht werden.

In einer weiteren Ausgestaltung der Erfindung ist das die Anlagefläche aufweisende Bauteil zumindest eines Kupplungselementes in Richtung der Kupplungsachse entgegen dem Kupplungsende dieses Kupplungselementes gegen eine Kraft verschiebbar.

Dadurch, daß dieses Element gegen eine Kraft verschiebbar ist, wird durch diese Kraft der notwendige Anpreßdruck zwischen den Anlageflächen geschaffen, um einen dichten Abschluß zu schaffen.

Die Bewerkstelligung des dichtenden Abschlusses ist somit, anders als bei einer Bajonettverriegelung, nicht mehr davon abhängig, wie weit die Bajonettverriegelung verdreht wird, sondern der Dichtdruck kann herstellerseitig mit einer ganz bestimmten Kraft voreingestellt werden, also unabhängig von den Gewohnheiten oder der Erfahrung der Handhabungsperson, was einen erheblichen Vorteil im Hinblick auf die Funktionssicherheit der miteinander verkuppelten Instrumente darstellt.

In einer weiteren Ausgestaltung der Erfindung ist der querverschiebbare Schieber in Richtung der verriegelnden Schiebestellung durch Federkraft beaufschlagt.

Diese Maßnahme hat den Vorteil, daß die Handhabung und die Betriebssicherheit noch dadurch weiter vereinfacht bzw. erhöht ist, daß der querverschiebbare Schieber in Richtung der verriegelnden Schiebestellung durch Federkraft beaufschlagt ist, also die Tendenz hat, sich selbst dahin zu bewegen. Somit ist ein Lösen der Verriegelung bspw. durch Erschütterungen oder versehentliches Berühren des Schiebers ausgeschlossen. Zum Bewerkstelligen der Kupplung muß der Schieber lediglich gegen die Kraft der Feder querverschoben werden, was bspw. durch einen Finger einer Hand einfach zu bewerkstelligen ist, so daß die beiden Kupplungselemente aneinandergefügt werden können, anschließend bewegt sich der Schieber von selbst aufgrund der Federkraft in die verriegelnde Stellung.

In einer weiteren Ausgestaltung der Erfindung ist der querverschiebbare Schieber in zumindest einer Endstellung arretierbar.

Diese Maßnahme hat nun den Vorteil, daß der Schieber in den beiden definierten Endstellungen arretiert ist, somit bei Handhabungen, bspw. beim Ablegen nach einem ersten Benutzen in dieser bestimmten Stellung verbleibt und nicht verschoben wird.

War bspw. eine Optik mit einem Arthroskopschaft gekuppelt und wurde die Kupplung dadurch gelöst, daß der querverschiebbare Schieber in seine offene freigebende Stellung verschoben wird, verbleibt der Schieber in dieser Stellung, auch nachdem die Optik zeitweilig beiseite gelegt wurde, um durch den Arthroskopschaft zwischenzeitlich ein anderes Instrument zu führen. Wird die Optik nach Beendigung dieses Vorganges wieder an den Arthroskopschaft angesetzt, ist sichergestellt, daß sich der Schieber nach wie vor in seiner offenen Stellung befindet. Dies erfolgt unabhängig davon, ob der Schieber am Arthroskopschaft oder an der Optik angeordnet ist.

Dies trägt erheblich zu der Vereinfachung der Handhabung und der Betriebssicherheit der Kupplung bei.

In einer weiteren Ausgestaltung der Erfindung erfolgt die Arretierung in der offenen Stellung des Schiebers und die Arretierung ist durch Anlegen des anderen Kupplungselementes lösbar.

Diese Maßnahme erhöht nun die Einfachheit der Handhabung der Kupplung deswegen beachtlich, da einerseits der Schieber, wie zuvor erwähnt, in dieser Endstellung arretiert ist und diese Arretierung durch das Anlegen des anderen Kupplungselementes gelöst wird. Steht diese Konstruktion noch in Zusammenhang mit dem zuvor erwähnten Merkmal des durch Federkraft beaufschlagten Schiebers, ist dieser Vorgang besonders einfach durchzuführen. Es muß nämlich nur das andere Kupplungselement angelegt werden, dadurch wird dann die Arretierung des Schiebers gelöst, und aufgrund der Kraft der Feder wird der Schieber automatisch in die sperrende Stellung verschoben. Beim Lösen der Verbindung muß dann lediglich der Schieber gegen die Kraft der Feder wieder aus der sperrenden in die offene Stellung verschoben werden, soweit bis er in der Endstellung wieder arretiert wird, ein Vorgang, der ohne hohe Aufmerksamkeit, bspw. durch einen Finger, einen Daumen oder dgl., durchgeführt werden kann, anschließend kann die Verbindung gelöst werden.

In dieser Kombination ist die Kupplung besonders handhabungsfreundlich.

In einer weiteren Ausgestaltung der Erfindung ist das Bauteil des Kupplungselementes, das in Richtung der Kupplungsachse verschiebbar ist, Teil der Arretierung.

Diese Maßnahme hat nun den Vorteil, daß dieses Bauteil mehrere Funktionen übernimmt, somit zu einer einfachen Konstruktion führt, die die Betriebssicherheit erhöht. Das mit Kraft beaufschlagte und in Richtung der Kupplungs- bzw. Längsachse der Schäfte bewegliche Bauteil dient dazu, um für den notwendigen dichtenden Anpreßdruck zwischen den Anlageflächen der miteinander zu verkuppelnden Instrumente zu sorgen. Diese Verschiebebewegung kann nun dazu herangezogen werden, um die Arretierung des Schiebers zu steuern. Wird also ein Kupplungselement des anzukuppelnden Instrumentes an dieses Bauteil angelegt und verriegelt dieses den Schieber, wird durch Verschieben dieses Bauteils die Verriegelung gelöst und der Schieber kann bewegt, ggf. bei der Ausgestaltung mit dem mit Federkraft beaufschlagten Schieber, dieser automatisch in die verriegelnde Stellung bewegt werden.

In einer weiteren Ausgestaltung der Erfindung ist am anderen Kupplungselement eine Hinterschneidung vorgesehen, in die Riegelbereiche des Schiebers in der riegelnden Schiebestellung eingreifen können.

Diese an sich bekannte Maßnahme der Hinterschneidung ist in Zusammenhang mit dem schlüssellochartigen Schieber konstruktiv besonders einfach und betriebssicher.

In einer weiteren Ausgestaltung der Erfindung springen am anderen Kupplungselement radial Laschen vor, hinter die Riegelbereiche des Schiebers in die Hinterschneidung eingreifen.

Diese ebenfalls an sich bekannte Maßnahme führt zu dem Vorteil, daß an sich schon bekannte und vorhandene Kupplungselemente mit dem nun vorgeschlagenen Kupplungselement mit dem querverschiebbaren Schieber verbunden werden können.

In einer weiteren Ausgestaltung der Erfindung geht der größere Öffnungsbereich der schlüssellochartigen Öffnung in einen kleineren Öffnungsbereich über, dessen seitliche Einengungen die Riegelbereiche des Schiebers bilden.

Diese Maßnahmen führen nicht nur zu fertigungstechnisch einfach herstellbaren Schiebern, sondern bilden auch Elemente ohne Nischen, die somit einfach zu reinigen und zu sterilisieren sind.

In einer weiteren Ausgestaltung der Erfindung sind die Anlageflächen der Kupplungselement als konische Flächen ausgebildet.

Diese an sich bekannte Maßnahme hat den erheblichen Vorteil, daß die beiden Kupplungselemente über relativ große Flächen aneinanderliegen, die sich selbst zentrieren, so daß schon geringe Anpreßdrücke ausreichen, um eine dichte Verbindung zwischen den Anlageflächen zu schaffen.

In einer weiteren Ausgestaltung der Erfindung ist der Schieber Teil eines querverschiebbaren Gehäuses.

Diese Maßnahme hat den Vorteil, daß das Gehäuse die "inneren" Kupplungselemente schützend umgibt, wobei das Gehäuse ein Bauelement ist, das ohne große Aufmerksamkeit von einem Finger einer Hand ertastet und bewegt werden kann.

In einer weiteren Ausgestaltung der Erfindung ist im Gehäuse eine sich in Verschieberichtung des Schiebers erstreckende Lauffläche vorgesehen, auf der ein Steuerelement des in Kupplungsrichtung verschiebbaren Bauteiles läuft.

Diese Maßnahme hat den Vorteil, daß durch konstruktiv einfache Maßnahmen das verschiebbare Bauteil definiert bewegt werden kann, bspw. um die zuvor erwähnte Verriegelung des Schiebers zu bewerkstelligen oder zu lösen.

In einer weiteren Ausgestaltung der Erfindung weist die Lauffläche zumindest an einem Ende eine Nische auf, in die das Steuerelement in einer Endstellung des Schiebers in Kupplungsrichtung einrückbar ist und dadurch den Schieber gegen Verschieben arretiert.

Dies stellt gleichermaßen eine einfach mechanisch zu bewerkstelligende Maßnahme dar, um die Arretierung des Schiebers zu bewerkstelligen.

In einer weiteren Ausgestaltung der Erfindung weist das in Kupplungsrichtung verschiebbare Bauteil einen etwa zylindrischen Abschnitt auf, dessen eines Ende eine als Innenkonus ausgebildete Anlagefläche aufweist, und um dessen Außenseite ist zumindest ein ringförmiges elastisches Element angeordnet, das das Bauteil in Richtung Kupplungsende des Kupplungselementes drückt.

Durch konstruktiv einfach zu bewerkstelligende Maßnahmen wird die konische Anlagefläche geschaffen und zugleich durch das deformierbare ringförmige elastische Element diejenige Kraft zur Verfügung gestellt, die notwendig ist, um den dichtenden Anpreßdruck in Kupplungsrichtung zu schaffen. Dadurch wird aber auch quer zur Kupplungsrichtung eine gewisse Elastizität geschaffen. Da dieses Bauteil die konische Anlagefläche trägt, ist dieser Dichtkonus quasi "schwimmend" gelagert und kann geringen Winkelveränderungen aus der Kupplungsachse heraus der an dieser konischen Fläche anliegenden Anlagefläche des anderen Instrumentes folgen, so daß eine optimale Dichtigkeit gewährt ist.

In einer weiteren Ausgestaltung der Erfindung stehen vom Bauteil zwei diametral gegenüberliegende, radial vorspringende Steuerzapfen vor, die auf der Lauffläche im Gehäuse des Schiebers liegen.

Diese Maßnahme hat den Vorteil, daß über die Steuerzapfen das Bauteil definiert entlang der Lauffläche im Gehäuse des Schiebers geführt werden kann, und in Verbindung mit dem ringförmigen elastischen Element, das um das Bauteil gelegt ist, immer ein entsprechender Anpreßdruck vorhanden ist, um die Steuerzapfen immer in Anlage mit der Lauffläche zu halten.

In einer weiteren Ausgestaltung der Erfindung ist das Bauteil in einem Führungselement aufgenommen, das wiederum im Gehäuse des Schiebers angeordnet ist.

Diese Maßnahme hat den Vorteil, daß das Bauteil zusammen mit dem Führungselement als kompakte Baueinheit ausgebildet und dementsprechend vormontiert werden kann, und dann geschützt im Gehäuse aufgenommen ist.

In einer weiteren Ausgestaltung ist das Führungselement fest mit einem Schaft eines Instrumentes verbunden, und das Gehäuse des Schiebers ist über das Führungselement geschoben, ferner sind diese Bauelemente über Führungsstangen, die durch das Gehäuse und das Führungselement reichen, querverschiebbar am Instrument angebracht.

Die Führungsstangen dienen nicht nur der Verbindung dieser beiden Bauelemente sondern gleichzeitig auch der Führung des Gehäuses, das den Schieber trägt, relativ zu dem Führungselement, das mit dem Schaft starr verbunden ist.

In einer weiteren Ausgestaltung sind die Führungsstangen als Schraubbolzen ausgeführt.

Diese Maßnahme hat den Vorteil, daß die Führungsstangen nicht nur der Führung sondern auch der lösbaren Montage dieser Bauelemente dienen.

In einer weiteren Ausgestaltung der Erfindung sind die Führungsstangen durch eine Zylinderöffnung in einer Wand des Führungselementes geführt, und es ist in der Öffnung eine die Führungsstange umgebende Schraubenfeder angeordnet, die sich an einem Ende an einer Schulter der Führungsstange und am anderen Ende an einem Ringbogen der Zylinderöffnung abstützt.

Diese Maßnahme hat nun den erheblichen Vorteil, daß durch konstruktiv sehr einfache und baulich geschützte Maßnahmen die durch Federkraft unterstützte Bewegung des querverschiebbaren Schiebers geschaffen wird.

In einer weiteren Ausgestaltung der Erfindung ist das Aneinanderlegen der beiden Kupplungselemente in der ersten Schiebestellung des Schiebers in einer beliebigen Drehstellung relativ zueinander durchführbar.

Diese Maßnahme hat den Vorteil, daß das Aneinanderlegen ohne besondere Aufmerksamkeit durchgeführt werden kann, da eine ganz bestimmte vorgegebene Relativstellung zwischen den aneinanderzufügenden Teilen nicht mehr notwendig ist. Bei Kupplungselementen mit kreisförmigem Querschnitt kann somit in jeder beliebigen Drehstellung miteinander verkuppelt werden.

In einer weiteren Ausgestaltung der Erfindung ist das Aneinanderlegen der beiden Kupplungselemente in der ersten Schiebestellung des Schiebers nur in einer bestimmten Drehstellung zueinander durchführbar.

Diese Maßnahme hat den Vorteil, daß, falls gewünscht ist, daß die beiden miteinander zu verbindenden medizinischen Instrumente in einer bestimmten Drehstellung zueinander gekuppelt werden sollen, diese Drehstellung schon beim Ansetzen erzielt wird. Dadurch entfällt ein nachträgliches Ausrichten nach Anbringen der Kupplung. Unter einer bestimmten Drehstellung können auch bestimmte mehrere Drehstellungen verstanden sein, beispielsweise um 180° zueinander verdrehte Drehstellungen, wenn es möglich ist, in diesen beiden Relativstellungen miteinander zu verkuppeln.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Explosionsdarstellung der wesentlichen Bauteile einer erfindungsgemäßen Kupplung;
- Fig. 2: einen Längsschnitt der erfindungsgemäßen Kupplung in einer ersten Betriebsstellung, in der die beiden Kupplungselemente aneinandergelegt, jedoch noch nicht miteinander verriegelt sind;
- Fig. 3: eine teilweise aufgebrochene perspektivische Darstellung der erfindungsgemäßen Kupplung in dem Betriebszustand von Fig. 2;
- Fig. 4: eine der Fig. 2 entsprechende Darstellung der erfindungsgemäßen Kupplung in verriegeltem Zustand,
- Fig. 5: eine der Darstellung von Fig. 3 entsprechende aufgebrochene perspektivische Darstellung des Betriebszustandes von Fig. 4;
- Fig. 6: eine der Darstellung von Fig. 3 ähnelnde aufgebrochene perspektivische Darstellung einer weiteren Ausgestaltungsform einer erfindungsgemäßen Kupplung;
- Fig. 7: eine Darstellung der Kupplung von Fig. 6 im Längsschnitt;
- Fig. 8: eine der Fig. 6 entsprechende Darstellung der Kupplung in einem Zwischenzustand, unmittelbar vor der Verriegelung;
- Fig. 9: eine der Fig. 7 entsprechende vereinfachte Schnittdarstellung der Stellung von Fig. 8;
- Fig. 10: eine der Fig. 6 entsprechende Darstellung im verriegelten Zustand; und
- Fig. 11: eine der Fig. 7 entsprechende Schnittdarstellung des verriegelten Zustandes, wie er in Fig. 10 gezeigt ist.

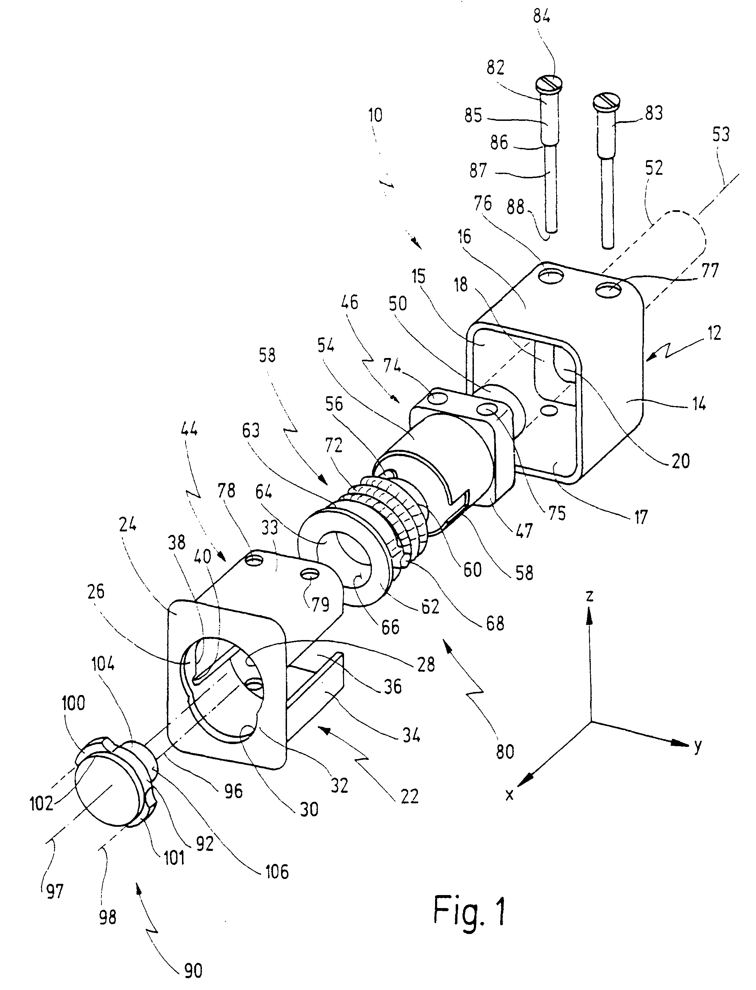

In den Fig. 1 bis 5 sind teilweise stark schematisiert diejenigen Bauelemente zweier medizinischer Instrumente 10 und 90 dargestellt, die notwendig sind, um die Verriegelung der beiden Instrumente 10 und 90 miteinander längs einer Schaft- 53 bzw. Kupplungsachse 97 zu bewerkstelligen.

Das in Fig. 1 in Explosionsdarstellung aufgezeigte Instrument 10 stellt ein proximales Ende eines Arthroskopschaftes dar, dessen rohrförmiger Schaft 52 mit gestrichelten Linien angedeutet ist.

Das Instrument 10 weist eine Kappe 12 auf, die zwei rechteckige, sich etwa parallel und im Abstand zueinander erstreckende Seitenwände 14 und 15 aufweist, die über sanfte Rundungen mit einer oberen Wand 16 bzw. einer unteren Wand 17 verbunden sind.

Die Kappe 12 ist über eine hintere Wand 18 abgeschlossen, in der ein Langloch 20 vorgesehen ist. Auf der dem Betrachter der Fig. 1 zugewandten Seite ist die Kappe 12 offen.

Es ist ein Zwischengehäuse 22 vorgesehen, das auf der dem Betrachter von Fig. 1 zugewandten vorderen Seite über eine Stirnplatte 24 abgeschlossen ist.

In der Stirnplatte 24 ist eine schlüssellochartigen Öffnung 26 ausgespart, die einen großen Öffnungsbereich 28 aufweist, der in einen kleinen Öffnungsbereich 30 übergeht, die jeweils eine etwa kreisförmige Kontur aufweisen. Diejenigen Bereiche der Stirnplatte 24, die um den kleinen Öffnungsbereich 30 herum angeordnet sind, werden als Riegelbereich 32 bezeichnet, deren Sinn und Zweck später in Zusammenhang mit der Funktionsweise der Kupplung erläutert werden.

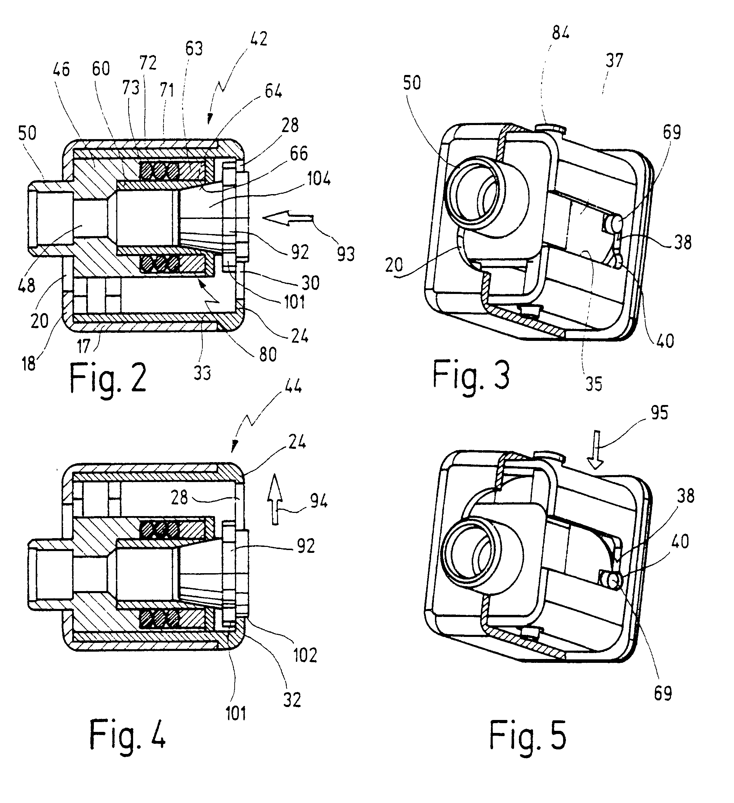

Von der Stirnplatte 24 springt etwa rechtwinklig eine gehäuseartige Wandung 33 vor, in deren gegenüberliegenden Seitenwänden 34 bzw. 35 je eine etwa U-förmige Ausnehmung 36 bzw. 37 ausgespart ist (siehe insbesondere die perspektivische Darstellung von Fig. 3 und 5).

Ein Boden der U-förmigen Ausnehmungen 36 bzw. 37 weist eine besonders konturierte Lauffläche 38 bzw. 39 auf, die an einem Ende eine Nische 40 bzw. 41 aufweist.

Die von der Stirnplatte 24 vorspringende Wandung 33 ist so bemaßt, daß deren Außenseite passend und eng an die Innenseite der Kappe 12 anliegend in diese eingeschoben werden kann, wie das insbesondere aus der Schnittdarstellung von Fig. 2 ersichtlich ist.

Ist das Zwischengehäuse 22 in die Kappe 12 eingeschoben, bildet sich insgesamt ein Gehäuse 42, wie es aus der Schnittdarstellung von Fig. 2 ersichtlich ist, das in Fig. 1 auf der dem Betrachter zugewandten Seite durch die Stirnplatte 24 mit der schlüssellochartigen Öffnung 26 auf der gegenüberliegenden Seite durch die hintere Wand 18 mit dem Langloch 20 abgeschlossen ist.

Ein in der perspektivischen Explosionsdarstellung von Fig. 1 zwischen der Kappe 12 und dem Zwischengehäuse 22 angeordnetes Führungselement 46 weist eine sich quer zur Schaftachse 53 erstreckende Wand 47 auf, die mittig eine Öffnung 48 (siehe Schnittdarstellung von Fig. 2) aufweist. In Richtung der hinteren Wand 18 der Kappe 12 springt ein Rohrflansch 50 vor, der sich im montierten Zustand (siehe Fig. 2) durch das Langloch 20 in der hinteren Wand 18 der Kappe 12 hindurch erstreckt. Am Rohrflansch 50 ist der Schaft 52 des Arthroskopes fest angebracht, der der Übersichtlichkeit halber in Fig. 1 nur durch gestrichelte Linien angedeutet ist.

Auf der dem Rohrflansch 50 gegenüberliegenden Seite der Wand 47 springt ein zylindrischer Abschnitt 54 vor, der diametral gegenüberliegende seitliche U-förmige Aussparungen 56 und 57 aufweist.

Der zylindrische Abschnitt 54 dient zur Aufnahme eines Bauteiles 58.

Das Bauteil 58 ist als Rohrstück 60 ausgebildet, wie das insbesondere aus der Schnittdarstellung von Fig. 2 ersichtlich ist.

Das Rohrstück 60 weist an dem der Stirnplatte 24 zugewandten Ende einen radialen Ringflansch 62 auf, der als Anschlag für einen über das Rohrstück geschobenen Ring 63 dient.

Auf Seiten des Ringflansches 62 weist das Rohrstück 60 an der Innenseite einen Innenkonus 64 auf, der als Anlagefläche 66 für einen entsprechenden Außenkonus 104 des anderen Instrumentes 90 dient.

Vom Ring 63 springen radial, diametral gegenüberliegende Steuerzapfen 68 bzw. 69 vor, deren radiale Länge so bemessen ist, daß diese sich durch die Aussparungen 56 und 57 in dem zylindrischen Abschnitt 54 hindurch bis in die Ausnehmungen 36 und 37 im Zwischengehäuse 22 erstrecken.

Über die Außenseite des Rohrstückes 60 des Bauteiles 58 sind ringförmige elastische Elemente in Form von drei O-Ringen 71, 72 und 73 geschoben, wobei der Übersichtlichkeit halber in Fig. 1 nur der mittlere O-Ring 72 bezeichnet ist.

In der Wand 47 des Führungselementes 46 sind durchgehende, sich quer zur Schaftachse bzw. Kupplungsachse 53 erstreckende Bohrungen 74 und 75 vorgesehen.

In der Kappe 12 sind in der oberen Wand 16 Löcher 76 und 77 und entsprechende, mit diesen fluchtende Löcher in der unteren Wand 17 vorgesehen.

Ebenso sind in der umlaufenden Wandung 33 des Zwischengehäuses 22 entsprechende Löcher 78, 79 bzw. bodenseitige, mit diesen Löchern fluchtende Löcher vorgesehen.

Im montierten Zustand sind die in der Explosionsdarstellung von Fig. 1 längs der Kupplungs- bzw. Schaftachse 53 auseinandergezogene Bauelemente ineinandergeschoben, wie das aus Fig. 2 ersichtlich ist.

In diesem montierten Zustand fluchten die Löcher 76, 77 der Kappe 12 mit den Bohrungen 74 und 75 der Wand 47 des Führungselementes 46 und mit den Löchern 78 und 79 des Zwischengehäuses 22. Durch diese fluchtenden Öffnungen werden Führungsstangen 82 und 83 in Form von Schraubenbolzen durchgeschoben.

Jede der Führungsstangen 82 und 83 weist einen Kopf 84, einen ersten zylindrischen Abschnitt 85, eine Schulter 86 und einen etwas dünneren zylindrischen Abschnitt 87 auf. Am unteren Ende des dünneren zylindrischen Abschnittes 87 ist ein Innengewinde 88 vorgesehen, in das von der Gegenseite eine entsprechende Schraube eingedreht werden kann.

Im montierten Zustand bildet der Zusammenbau aus Bauteil 58 und Führungselement 46 ein Kupplungselement 80. Der Zusammenbau aus Kappe 12 und Zwischengehäuse 22 bildet einen quer zur Schaftachse bzw. Kupplungsachse 53 verschiebbaren Schieber 44, der als Verriegelung dient. Dazu ist der Abstand zwischen der oberen Wand 16 und der unteren Wand 17 der Kappe größer als die Bauhöhe der Wand 47 des Führungselementes 46.

Betrachtet man das in Fig. 1 dargestellte Koordinatensystem, so erstreckt sich die x-Achse in Richtung der Kupplungs- bzw. Schaftachse 53. Die Führungsstangen 82 und 83 erstrecken sich längs der z-Achse, d.h. der Schieber 44 kann längs der z-Achse relativ zum Kupplungselement 80 verschoben werden.

Das Kupplungselement 80 dient dazu, mit einem Kupplungselement 92 eines weiteren Instrumentes 90 verbunden zu werden.

Im dargestellten Ausführungsbeispiel ist das Instrument 90 eine Optik, die mit dem Arthroskopschaft (Instrument 10) dichtend verbunden werden soll.

Das Instrument 90 weist dazu einen vom Kupplungselement 92 vorspringenden Schaft 96 auf, der durch das Kupplungselement 80 hindurch und durch den Schaft 52 des Arthroskopes bis an dessen patientennahes Ende reicht. Dem Schaft 96 gegenüberliegend springt vom Kupplungselement 92 ein weiterer Bauabschnitt 98 der Optik vor, der bspw. das Linsensystem enthält, der der Übersichtlichkeit halber hier nur angedeutet ist.

Das Kupplungselement 92 weist radial vorstehende Laschen 100, 101 auf, deren Radialerstreckung so ausgebildet ist, daß das Kupplungselement 92 durch den großen Öffnungsbereich 28 der schlüssellochartigen Öffnung 26 hindurchgeschoben werden kann, nicht jedoch durch den kleinen Öffnungsbereich 30.

Von distal nach proximal gesehen ist hinter den Laschen 100, 101 eine Hinterschneidung 102 vorgesehen, in die die Riegelbereiche 32 der Stirnplatte 24, die den kleinen Öffnungsbereich 30 umgrenzen, eingreifen können.

Die Laschen 100 und 101 können auch als umlaufender Ringflansch ausgebildet sein, dies ist aber nicht unbedingt notwendig, deren umfängliches Maß muß so gewählt werden, daß in jeder beliebigen Drehstellung des Kupplungselementes 92 auf jeden Fall ein sperrender Eingriff durch die Riegelbereiche 32 des kleinen Öffnungsbereiches 30 der schlüssellochartigen Öffnung 26 stattfindet, falls das Kupplungselement 92 mit dem kleineren Öffnungsbereich 30 fluchtet.

Wie aus der Schnittdarstellung von Fig. 2 zu entnehmen, sind die drei elastischen O-Ringe 71, 72, 73 in einem Ringraum aufgenommen, so daß das Bauteil 58 in Richtung der Kupplungs- bzw. Schaftachse 53 federnd gelagert ist und gegen die Rückfederungskraft der elastischen O-Ringe 71, 72, 73 in dieser Richtung etwas verschieblich ist.

In der in Fig. 2 dargestellten Stellung befindet sich der Schieber 44 in der offenen, nicht sperrenden Stellung, d.h. der Innenkonus 64 des Bauteiles 58 fluchtet mit dem großen Öffnungsbereich 28 der schlüsselförmigen Öffnung 26. In dieser Stellung kann nun das Kupplungselement 92 des mit dem Instrument 10 zu kuppelnden Instrumentes 90 durch den großen Öffnungsbereich 28 der schlüssellochartigen Öffnung 26 hindurchgeschoben werden, wie dies in Fig. 2 durch einen Pfeil 93 dargestellt ist. Dabei kommt der Außenkonus 104 des Kupplungselementes 92 zur Anlage mit dem Innenkonus 64 des Bauteiles 58. Die Bemessung ist so, daß die Laschen 100, 101 gerade durch die Öffnung 26 hindurchgetreten sind.

Wie aus Fig. 3 zu ersehen, liegen die radial vorspringenden Steuerzapfen 68 und 69 des Bauteils 58 an einem, in der Darstellung von Fig. 3 oberen Ende der Lauffläche 38 an, wobei ein Anpreßdruck durch die O-Ringe 71 bis 73 geschaffen wird.

Der Innenkonus 64 des Bauteiles 58 ist somit in x-Richtung vorgespannt. Das Verriegeln der über die Anlageflächen 96 und 106 der beiden Kupplungselemente 80 und 92 aneinanderliegenden Instrumente 10 und 90 wird nun dadurch bewerkstelligt, daß der Schieber 44 in z-Richtung verschoben wird, wie das in Fig. 4 durch einen Pfeil 94 angedeutet ist. Dabei schiebt sich der kleine Öffnungsbereich 30 der schlüssellochartigen Öffnung 96 bzw. die diesen kleinen Öffnungsbereich 30 umgebenden Riegelbereiche 32 der Stirnplatte 24 in die Hinterschneidung 102 hinter den Laschen 100 und 101 und sperrt mechanisch ein Lösen der Instrumente 10 und 90 voneinander.

Bei diesem Querverschieben des Schiebers 44 haben sich die Steuerzapfen 68 und 69, wie das aus der Bildfolge von Fig. 3 zu Fig. 5 ersichtlich ist, längs der Lauffläche 38 bewegt und sind in die Nische 40 eingerückt. Dieses Einrücken wird durch den Druck der etwas zusammengepreßten O-Ringe 71 bis 73 bewerkstelligt. Dieses Einrücken bzw. dieser Druck sorgt auch dafür, daß nunmehr, im verriegelten Zustand, das Bauteil 58 bzw. dessen Innenkonus 64 fest gegen den Außenkonus 104 des Kupplungselementes 92 gedrückt wird.

Dieser Anpreßdruck wirkt somit erst dann, wenn die Steuerzapfen 68 bzw. 69 in die Nischen 40, 41 eingerückt sind, also zu einem Zeitpunkt, zu dem bereits ein Abziehen des Kupplungselementes 92 gesperrt ist, da bereits die Riegelbereiche 32 der Stirnplatte 24 in die Hinterschneidung 102 eingreifen. Das bedeutet, beim Kuppeln müssen die beiden Kupplungselemente 80 und 92 nur aneinandergelegt werden, wobei es auf die relative Drehstellung dieser Teile nicht ankommt. Der Schieber 44 kann bspw. mit einem Finger einer menschlichen Hand einfach verschoben werden, danach wirkt der zusätzliche Anpreßdruck durch die O-Ringe 71, 72, 73, so daß ein dichter Sitz der aneinandergefügten und verriegelten Instrumente 10 und 90 gewährleistet ist. Die "schwimmende" Lagerung des Bauteiles 58 in dem Führungselement 46 erlaubt auch ein, wenn auch nur in geringem Maße, Abkippen um die y-Achse, ohne daß der dichtende Sitz zwischen den Anlageflächen 66 und 106 beeinträchtigt wird.

Zum Lösen der Kupplung wird durch Druck auf den Schieber 44, wie das durch einen Pfeil 95 in Fig. 5 angezeigt ist, die Verriegelung wieder gelöst.

Dazu ist der Übergang von der Nische 40 zur Lauffläche 38 so ausgebildet, daß durch einen sanften Handdruck auf den Schieber der Steuerzapfen 69 aus der Nische 40 ausrückt.

Dabei wird das Bauteil 58 etwas in -x-Richtung verschoben, somit der Anpreßdruck der Anlageflächen 96 und 106 wieder gelöst, das Kupplungselement 92 kann nun aus dem Gehäuse 42 des Schiebers 44 wieder über den größeren Öffnungsbereich 28 abgezogen werden.

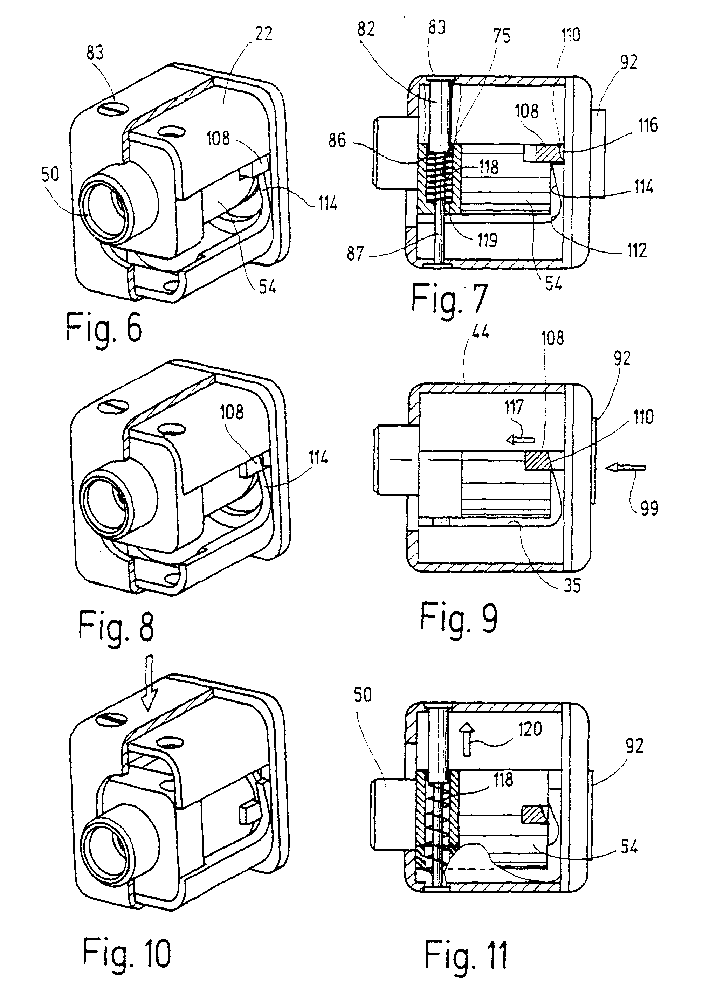

In den Fig. 6 bis 11 ist eine weitere Ausgestaltung eines Kupplungselementes 80 eines Instrumentes 10 dargestellt, dessen wesentliche Bauteile identisch zu den in Zusammenhang mit Fig. 1 bis 5 beschriebenen Bauteilen sind, so daß diese Bezugszeichen übernommen werden und nachfolgend lediglich die Abweichungen beschrieben werden.

Eine Abweichung besteht in der Ausgestaltung der Lauffläche 112 in der seitlichen Ausnehmung 35 im Zwischengehäuse 22.

Der in Fig. 6 und 7 dargestellte zusammengesetzte Zustand entspricht dem zuvor in Zusammenhang mit Fig. 2 und 3 beschriebenen Bauzustand, d.h. der große Öffnungsbereich 28 der schlüssellochartigen Öffnung 26 fluchtet mit dem Innenkonus 64 des Bauteiles 58. Die von diesem Bauteil 58 vorstehenden Steuerzapfen 108 sind nun so ausgebildet, daß sie eine der Kupplungsstelle zugewandte Schräge 110 aufweisen.

Die Lauffläche 112 weist eine komplementäre Schräge 114 auf, und weist in der Darstellung von Fig. 7 an ihrem oberen Ende eine Nische 116 auf, in die der Steuerzapfen 108 eintreten kann.

Das bedeutet, daß der in die Nische 116 eingerückte Steuerzapfen 108 den Schieber in seiner offenen, nicht verriegelnden Stellung sperrt.

Ferner sind im Gegensatz zu der in Fig. 1 bis 5 dargestellten Ausführung in den Bohrungen 74 und 75 der Wand 47 des Führungselementes, durch die die Führungsstangen 82 und 83 hindurchreichen, die Führungsstangen 82 und 83 umrundende Schraubenfedern 118 angeordnet.

Die Schraubenfedern 118 stützen sich einerseits an der Schulter 86 der Führungsstangen 82 bzw. 83, am gegenüberliegenden Ende auf einem Ringboden 119 der sacklochartigen Bohrungen 74 bzw. 75 ab.

Der dünnere zylindrische Abschnitt 87 der Führungsstangen 82 bzw. 83 reicht durch den Ringboden 119 hindurch, und in dessen Innengewinde (hier nicht bezeichnet) ist eine entsprechende (hier ebenfalls nicht bezeichnet) Schraube eingedreht. Die Federn 118 sind auf Druck vorgespannt.

Aufgrund des sperrenden Eingriffs des Steuerzapfens 108 in die Nische 116 kann der Schieber 44 in der in Fig. 6 und 7 dargestellten Position nicht längs der z-Achse verschoben werden.

Wird nun das Kupplungselement 92 des Instrumentes 90 durch die schlüssellochartige Öffnung 26 eingeschoben, trifft dessen Außenkonus 104 auf den Innenkonus 64 des Bauteiles 58. Ein sanftes Eindrücken, wie das in Fig. 9 durch einen Pfeil 99 dargestellt ist, verursacht, daß das Bauteil 58 gegen die Federkraft der O-Ringe 71 bis 73 etwas in -x-Richtung verschoben wird.

Dabei bewegt sich der Steuerzapfen 108 aus der Nische 116 heraus, wie das in Fig. 9 durch einen Pfeil 117 dargestellt ist.

Ist der Steuerzapfen 108 aus der Nische 116 ausgerückt, kann nun der Schieber 44 in die sperrende Stellung verschoben werden, wie sie in Fig. 10 und 11 dargestellt ist, wobei dies automatisch durch die Kraft der Federn 118 erfolgt, wie das in Fig. 11 durch einen Pfeil 120 dargestellt ist.

Aufgrund der Schräge 114 der Lauffläche 112 kann nunmehr, aufgrund des Druckes der O-Ringe 71 bis 73 das Bauteil 58 sich wieder geringfügig in x-Richtung bewegen, so daß dann wieder für den notwendigen Anpreßdruck zwischen den Anlageflächen 66 und 106 gesorgt ist.

Beim Anlegen des Instrumentes 90 an das Instrument 10, was wieder in jeder beliebigen Winkeldrehstellung zueinander erfolgen kann, muß lediglich ein gewisser Anpreßdruck ausgeübt werden, was bspw. dadurch geschehen kann, daß eines der Instrumente zwischen Zeige- und Mittelfinger ergriffen und das andere mit dem Daumen derselben Hand auf dieses zugeschoben wird. Ist der Steuerzapfen 108 aus der Nische 116 ausgerückt, schnappt der Schieber 44 aufgrund der Kraftfedern 118 von selbst in die sperrende Stellung und verbleibt dort.

Zum Lösen der Kupplung muß dann der Schieber 44 entgegen die Kraft der Federn 118 in -z-Richtung gedrückt werden, wobei die gleichsinnigen Neigungen der Schräge 110 des Steuerzapfens 108 und der Schräge 114 der Lauffläche 112 ein geringfügiges Verschieben des Bauteiles 58 in -x-Richtung gegen die Preßkraft der O-Ringe 71 bis 73 verursachen, bis der Steuerzapfen 108 vor der Öffnung der Nische 160 zum Liegen kommt und dann in diese aufgrund der Federkraft der O-Ringe 71 bis 73 hinein schnappt, somit eine Rückkehr des Schiebers 44 mechanisch sperrt.

## Patentansprüche

1. Kupplung zum dichten Verbinden zweier schaftartiger medizinischer Instrumente (10, 90) längs deren Schaftachse (53, 97), mit einem an einem ersten Instrument (10) angeordneten, eine Anlagefläche (66) aufweisendes Kupplungselement (80), an dessen Anlagefläche (66) eine Anlagefläche (106) eines Kupplungselementes (92) des zweiten Instrumentes (90) längs einer Kupplungsachse (53) anlegbar ist, und mit einer Verriegelung zum lösbaren Verriegeln der beiden Kupplungselemente (80, 92) miteinander, wobei die Anlageflächen (66, 106) dichtend aneinanderliegen,
**dadurch gekennzeichnet, daß**
die Verriegelung einen quer zur Kupplungsachse (53) verschiebbaren Schieber (44) aufweist, der an einem der beiden Kupplungselemente (80) querverschiebbar angeordnet ist und der einen größeren Öffnungsbereich (28) und einen kleineren Öffnungsbereich (30) aufweist, wobei der verschiebbare Schieber (44) in einer ersten Schiebestellung das Anlegen der beiden Kupplungselemente (80, 92) durch den größeren Öffnungsbereich (28) aneinander ermöglicht und in einer zweiten riegelnden Schiebestellung die beiden Kupplungselemente (80, 92) in dem kleineren Öffnungsbereich (30) mechanisch verriegelt, und daß ein die Anlagefläche (66) aufweisenden Bauteil (58) zumindest eines Kupplungselementes (80) in Richtung der Kupplungsachse (53) verschiebbar ist.

2. Kupplung nach Anspruch 1, **dadurch gekennzeichnet, daß** das die Anlagefläche (66) aufweisende Bauteil (58) eines Kupplungselementes (80) entgegen dem Kupplungsende dieses Kupplungselementes (80) gegen eine Kraft verschiebbar ist.

3. Kupplung nach Anspruch 1 oder 2, **dadurch gekennzeichnet, daß** der querverschiebbare Schieber (44) in Richtung der verriegelnden Schiebestellung durch Federkraft beaufschlagt ist.

4. Kupplung nach einem der Ansprüche 1 bis 3, **dadurch gekennzeichnet, daß** der querverschiebbare Schieber (44) in zumindest einer Endstellung arretierbar ist.

5. Kupplung nach Anspruch 4, **dadurch gekennzeichnet, daß** die Arretierung in der offenen Stellung des Schiebers (44) erfolgt, und daß durch das Anlegen des anderen Kupplungselementes (92) die Arretierung lösbar ist.

6. Kupplung nach Anspruch 4 oder 5, **dadurch gekennzeichnet, daß** das Bauteil (58) des Kupplungselementes (80), das in Richtung der Kupplungsachse (53) verschiebbar ist, Teil der Arretierung ist.

7. Kupplung nach einem der Ansprüche 1 bis 6, **dadurch gekennzeichnet, daß** am anderen Kupplungselement (92) eine Hinterschneidung (102) vorgesehen ist, in die Riegelbereiche (32) des Schiebers (44) in der riegelnden Schiebestellung eingreifen.

8. Kupplung nach Anspruch 7, **dadurch gekennzeichnet, daß** am anderen Kupplungselement (92) radiale Laschen (100, 101) vorspringen, hinter die die Riegelbereiche (32) des Schiebers (44) in die Hinterschneidung (102) eingreifen.

9. Kupplung nach Anspruch 7 oder 8, **dadurch gekennzeichnet, daß** der größere Öffnungsbereich (28) der schlüssellochartigen Öffnung (26) in einen kleineren Öffnungsbereich (30) übergeht, dessen seitliche Einengungen die Riegelbereiche (32) des Schiebers (44) bilden.

10. Kupplung nach einem der Ansprüche 1 bis 9, **dadurch gekennzeichnet, daß** die Anlageflächen (66, 106) der Kupplungselemente (80, 92) als konische Flächen ausgebildet sind.

11. Kupplung nach einem der Ansprüche 1 bis 10, **dadurch gekennzeichnet, daß** der Schieber (44) Teil eines querverschiebbaren Gehäuses (42) ist.

12. Kupplung nach Anspruch 11, **dadurch gekennzeichnet, daß** im Gehäuse (42) eine sich in Verschieberichtung des Schiebers (44) erstreckende Lauffläche (38, 39; 112) vorgesehen ist, auf der ein Steuerelement (68, 69; 108) des in Kupplungsrichtung verschiebbaren Bauteiles (58) läuft.

13. Kupplung nach Anspruch 12, **dadurch gekennzeichnet, daß** die Lauffläche (38, 39; 108) zumindest an einem Ende eine Nische (40, 41; 116) aufweist, in die das Steuerelement (68, 69; 108) in einer Endstellung des Schiebers in Kupplungsrichtung (53) einrückbar ist, und dadurch den Schieber (44) gegen Verschieben quer zur Kupplungsrichtung (53) arretiert.

14. Kupplung nach einem der Ansprüche 1 bis 13, **dadurch gekennzeichnet, daß** das in Kupplungsrichtung (53) verschiebbare Bauteil (58) einen etwa zylindrischen Abschnitt (60) aufweist, dessen eines Ende eine als Innenkonus (64) ausgebildete Anlagefläche (66) aufweist, daß um dessen Außenseite zumindest ein ringförmiges elastisches Element (71 bis 73) angeordnet ist, das das Bauteil (58) in Richtung Kupplungsende des Kupplungselements (80) drückt.

15. Kupplung nach einem der Ansprüche 12 bis 14, **dadurch gekennzeichnet, daß** am Bauteil (58) zumindest zwei diametral gegenüberliegende, radial vorspringende Steuerzapfen (68, 69; 108) vorgesehen sind, die auf der Lauffläche (38, 39; 112) im Gehäuse (42) des Schiebers (44) liegen.

16. Kupplung nach einem der Ansprüche 11 bis 15, **dadurch gekennzeichnet, daß** das Bauteil (58) in einem Führungselement (46) aufgenommen ist, das wiederum im Gehäuse (42) des Schiebers (44) angeordnet ist.

17. Kupplung nach Anspruch 16, **dadurch gekennzeichnet, daß** das Führungselement (46) fest mit einem Schaft (52) des Instrumentes (10) verbunden ist, daß das Gehäuse (42) des Schiebers (44) über das Führungselement (46) geschoben ist, und daß diese Bauelemente über Führungsstangen (83, 84), die durch Gehäuse (42) und Führungselement (46) reichen, querverschiebbar am Instrument (10) angebracht sind.

18. Kupplung nach Anspruch 17, **dadurch gekennzeichnet, daß** die Führungsstangen (83, 84) als Schraubbolzen ausgeführt sind.

19. Kupplung nach Anspruch 17 oder 18, **dadurch gekennzeichnet, daß** die Führungsstangen (83, 84) durch eine Zylinderöffnung in einer Wand (47) des Führungselementes (46) geführt sind, daß in den Zylinderöffnungen die Führungsstangen (83, 84) umgebende Schraubenfedern (118) angeordnet sind, die sich an einem Ende an einer Schulter (86) der Führungsstange (83, 84) und am anderen Ende an einem Ringboden (119) der Zylinderöffnungen (74, 75) abstützen.

20. Kupplung nach einem der Ansprüche 1 bis 19, **dadurch gekennzeichnet, daß** das Aneinanderlegen der beiden Kupplungselemente (80, 92) in der ersten Schiebestellung des Schiebers (44) in einer beliebigen Drehstellung relativ zueinander durchführbar ist.

21. Kupplung nach einem der Ansprüche 1 bis 19, **dadurch gekennzeichnet, daß** das Aneinanderlegen der beiden Kupplungselemente in der ersten Schiebestellung (Fig. 2) des Schiebers (44) nur in einer bestimmten Drehstellung zueinander durchführbar ist.

## Claims

1. Coupling for leakproof connection of two shaft-like medical instruments (10, 90) along their shaft axis (53, 57), with a coupling element (80) arranged on a first instrument (10) and having a contact surface (66) against whose contact surface (66) a contact surface (106) of a coupling element (92) of the second instrument (90) can be applied along a coupling axis (53); and with an interlock system for releasable interlocking the two coupling elements (80, 92) to one another, the contact surfaces (66, 106) resting against one another in leakproof fashion, **characterized in that** the interlock system has a slider (44) which is displaceable transversely to the coupling axis (53) and which is arranged in transversely displaceable fashion on one of the two coupling elements (80), and having a larger opening region (28) and a smaller opening region (30), wherein the slidable slider (44) allows, in a first sliding position, to apply the two coupling elements (80, 92) across the larger opening region (28) one to another and, in a second locking sliding position, locks the two coupling elements (80, 92) in the smaller opening region (30) mechanically, and **in that** a component (58) having the contact surface (66) of at least one coupling element (80) is displaceable in the direction of the coupling axis (53).

2. Coupling of claim 1, **characterized in that** the component (58) having the contact surface (66) of at least one coupling element (80) is displaceable, against a force, toward the coupling end of said coupling element (80).

3. Coupling of claims 1 or 2, **characterized in that** the transversely displaceable slider (44) is acted upon by spring force in the direction of the interlocking slide position.

4. Coupling of anyone of claims 1 through 3, **characterized in that** the transversely displaceable slider (44) can be retained in at least one end position.

5. Coupling of claim 4, **characterized in that** the retention is accomplished in the open position of the slider (44), and **in that** the retention is releasable by applying the other coupling element (92).

6. Coupling of claims 4 or 5, **characterized in that** the component (58) of the coupling element (80) that is displaceable in the direction of the coupling axis (53) is part of the retention system.

7. Coupling of anyone of claims 1 through 6, **characterized in that** an undercut (102) in which the locking regions (32) of the slider (44) can engage in the locking slide position is provided on the other coupling element (92).

8. Coupling of claim 7, **characterized in that** radial tabs (100, 101), behind which the locking regions (32) of the slider (44) engage into the undercut (102), project from the other coupling element (92).

9. Coupling of claims 7 or 8, **characterized in that** the larger opening region (28) of the keyhole-shaped opening (26) transitions into a smaller opening region (30) whose lateral constrictions constitute the locking regions (32) of the slider (44).

10. Coupling of anyone of claims 1 through 9, **characterized in that** the contact surfaces (66, 106) of the coupling elements (80, 92) are configured as conical surfaces.

11. Coupling of anyone of claims 1 through 10, **characterized in that** the slider (44) is part of a transversely displaceable housing (42).

12. Coupling of claim 11, **characterized in that** a guiding surface (38, 39; 112) which extends in the displacement direction of the slider (44), and on which a control element (68, 69; 108) of the component (58) that can be displaced in the coupling direction is guided, is provided in the housing (42).

13. Coupling of claim 12, **characterized in that** the guiding surface (38, 39; 108) has at least at one end a recess (40, 41; 116) into which, when the slider is in one end position, the control element (68, 69; 108) can be engaged in the coupling direction (53) and thereby retains the slider (44) nondisplaceably transversely to the coupling direction (53).

14. Coupling of anyone of claims 1 through 13, **characterized in that** the component (58) that is displaceable in the coupling direction (53) has an approximately cylindrical segment (60) whose one end has a contact surface (66) configured as an internal taper (64), and around whose exterior is arranged at least one annular elastic element (71 through 73) which presses the component (58) in the direction of the coupling end of the coupling element (80).

15. Coupling of anyone of claims 12 through 14, **characterized in that** at least two diametrically opposite and radially projecting control pins (68, 69; 108), which rest on the guiding surface (38, 39; 112) in the housing (42) of the slider (44), are provided on the component (58).

16. Coupling of anyone of claims 11 through 15, **characterized in that** the component (58) is received in a guide element (46) which in turn is arranged in the housing (42) of the slider (44).

17. Coupling of claim 16, **characterized in that** the guide element (46) is connected immovably to a shaft (52) of an instrument (10); **in that** the housing (42) of the slider (44) is slid over the guide element (46); and **in that** these constituents are mounted on the instrument (10) in transversely displaceable fashion via guide rods (83, 84) which extend through the housing (42) and the guide element (46).

18. Coupling of claim 17, **characterized in that** the guide rods (83, 84) are embodied as threaded studs.

19. Coupling of claims 17 or 18, **characterized in that** the guide rods (83, 84) are guided through a cylindrical opening in one wall (47) of the guide element (46), and **in that** there are arranged in the cylindrical openings helical springs (118) surrounding the guide rods (83, 84), which brace at one end against a shoulder (86) of the guide rod (83, 84) and at the other end against an annular base (119) of the cylindrical openings (74, 75).

20. Coupling of anyone of claims 1 through 19, **characterized in that** the application of the two coupling elements (80, 92) against one another, with the slider (44) in the first slide position can be accomplished in any rotational position relative to one another.

21. Coupling of anyone of claims 1 through 19, **characterized in that** the application of the two coupling elements against one another, with the slider (44) in the first slide position, can be accomplished only in a specific rotational position with respect to one another.

## Revendications

1. Accouplement pour le raccordement étanche de deux instruments médicaux allongés (10, 90) le long de leur axe de tige (53, 97), avec un élément d'accouplement (80) disposé sur un premier instrument (10), comportant une surface d'appui (66), contre la surface d'appui (66) duquel une surface d'appui (106) d'un élément d'accouplement (92) du second instrument (90) peut être posée le long d'un axe d'accouplement (53), et avec un dispositif de verrouillage pour le verrouillage amovible des deux éléments d'accouplement (80, 92) l'un avec l'autre, les surfaces d'appui (66, 106) étant placées l'une à côté de l'autre de manière étanche, **caractérisé en ce que**
le dispositif de verrouillage comporte un coulisseau (44) déplaçable transversalement à l'axe d'accouplement (53), qui est placé de manière déplaçable transversalement sur l'un des deux éléments d'accouplement (80) et qui comporte une grande zone d'ouverture (28) et une petite zone d'ouverture (30), moyennant quoi, dans une première position de déplacement, le coulisseau déplaçable (44) permet de poser les deux éléments d'accouplement (80, 92) l'un contre l'autre par la grande zone d'ouverture (28) et, dans une seconde position de déplacement verrouillante, verrouille mécaniquement les deux éléments d'accouplement (80, 92) dans la petite zone d'ouverture (30), et **en ce qu'**un élément de construction (58) d'au moins un élément d'accouplement (80) comportant la surface d'appui (66) est déplaçable dans la direction de l'axe d'accouplement (53).

2. Accouplement selon la revendication 1, **caractérisé en ce que** l'élément de construction (58) d'un élément d'accouplement (80) comportant la surface d'appui (66) est déplaçable contre une force à l'opposé de l'extrémité d'accouplement de cet élément d'accouplement (80).

3. Accouplement selon la revendication 1 ou 2, **caractérisé en ce que** le coulisseau (44) déplaçable transversalement est soumis à une force de ressort dans la direction de la position de déplacement verrouillante.

4. Accouplement selon l'une quelconque des revendications 1 à 3, **caractérisé en ce que** le coulisseau (44) déplaçable transversalement est blocable dans au moins une position extrême.

5. Accouplement selon la revendication 4, **caractérisé en ce que** le blocage du coulisseau (44) se produit dans la position ouverte, et **en ce que** le dispositif de blocage peut être détaché par la pose de l'autre élément d'accouplement (92).

6. Accouplement selon la revendication 4 ou 5, **caractérisé en ce que** l'élément de construction (58) de l'élément d'accouplement (80) qui est déplaçable dans la direction de l'axe d'accouplement (53) fait partie du dispositif de blocage.

7. Accouplement selon l'une quelconque des revendications 1 à 6, **caractérisé en ce qu'**une contre-dépouille (102), dans laquelle s'engrènent des zones de verrouillage (32) du coulisseau (44) dans la position de déplacement verrouillante, est prévue sur l'autre élément d'accouplement (92).

8. Accouplement selon la revendication 7, **caractérisé en ce que** des colliers de fixation radiaux (100, 101), derrière lesquels s'engrènent les zones de verrouillage (32) du coulisseau (44) dans la contre-dépouille (102), font saillie sur l'autre élément d'accouplement (92).

9. Accouplement selon la revendication 7 ou 8, **caractérisé en ce que** la grande zone d'ouverture (28) de l'ouverture en forme de trou de serrure (26) se confond avec une petite zone d'ouverture (30) dont les rétrécissements latéraux forment les zones de verrouillage (32) du coulisseau (44).

10. Accouplement selon l'une quelconque des revendications 1 à 9, **caractérisé en ce que** les surfaces d'appui (66, 106) de l'élément d'accouplement (80, 92) sont configurées comme des surfaces coniques.

11. Accouplement selon l'une quelconque des revendications 1 à 10, **caractérisé en ce que** le coulisseau (44) fait partie d'un boîtier déplaçable transversalement (42).

12. Accouplement selon la revendication 11, **caractérisé en ce qu'**il est prévu dans le boîtier (42) une surface de roulement (38, 39 ; 112) s'étendant, dans la direction de déplacement du coulisseau (44), sur laquelle roule un élément de commande (68, 69 ; 108) de l'élément de construction (58) déplaçable dans la direction de l'accouplement.

13. Accouplement selon la revendication 12, **caractérisé en ce que** la surface de roulement (38, 39 ; 112) comporte, au moins à une extrémité, une niche (40, 41 ; 116), dans laquelle l'élément de commande (68, 69 ; 108) est engrené dans une position extrême du coulisseau dans la direction de l'accouplement (53), et bloque ainsi le coulisseau (44) contre un déplacement transversal à la direction de l'accouplement (53).

14. Accouplement selon l'une quelconque des revendications 1 à 13, **caractérisé en ce que** l'élément de construction (58) déplaçable dans la direction de l'accouplement (53) comporte un tronçon à peu près cylindrique (60) dont une extrémité comporte une surface d'appui (66) configurée comme un cône intérieur, **en ce qu'**un élément élastique annulaire (71 à 73), qui comprime l'élément de construction (58) dans la direction de l'extrémité d'accouplement de l'élément d'accouplement (80), est placé autour de la face externe de celui-ci.

15. Accouplement selon l'une quelconque des revendications 12 à 14, **caractérisé en ce qu'**au moins deux tourillons de commande (68, 69 ; 108) diamétralement opposés, faisant saillie dans le sens radial, qui sont placés sur la surface de roulement (38, 39 ; 112) dans le boîtier (42) du coulisseau (44), sont prévus dans l'élément de construction (58).

16. Accouplement selon l'une quelconque des revendications 11 à 15, **caractérisé en ce que** l'élément de construction (58) est logé dans un élément de guidage (46) qui est placé, lui aussi, dans le boîtier (42) du coulisseau (44).

17. Accouplement selon la revendication 16, **caractérisé en ce que** l'élément de guidage (46) est raccordé fixement à une tige (52) de l'instrument (10), **en ce que** le boîtier (42) du coulisseau (44) est déplacé par l'élément de guidage (46) et **en ce que** ces éléments de construction sont montés de manière déplaçable transversalement sur l'instrument (10) par l'intermédiaire de tiges de guidage (83, 84) qui s'étendent à travers le boîtier (42) et l'élément de guidage (46).

18. Accouplement selon la revendication 17, **caractérisé en ce que** les tiges de guidage (83, 84) sont réalisées comme des boulons filetés.

19. Accouplement selon la revendication 17 ou 18, **caractérisé en ce que** les tiges de guidage (83, 84) sont guidées à travers un orifice de cylindre dans une paroi (47) de l'élément de guidage (46), **en ce que** des ressorts à boudin (118) entourant les tiges de guidage (83, 84), qui s'appuient à une extrémité sur un épaulement (86) de la tige de guidage (83, 84) et à l'autre extrémité sur un fond annulaire (119) des orifices de cylindre (74, 75), sont placés dans les orifices de cylindre.

20. Accouplement selon l'une quelconque des revendications 1 à 19, caractérisé en ce le placement des deux éléments d'accouplement (80, 92) l'un à côté de l'autre dans la première position de déplacement du coulisseau (44) est réalisable dans n'importe quelle position de rotation l'un par rapport à l'autre.

21. Accouplement selon l'une quelconque des revendications 1 à 19, caractérisé en ce le placement des deux éléments d'accouplement l'un à côté de l'autre dans la première position de déplacement (figure 2) du coulisseau (44) n'est réalisable que dans une certaine position de rotation l'un par rapport à l'autre.
